# FASCICULE DE BREVET EUROPEEN

(11) **EP 2 938 286 B1**
(45) Date de publication et mention de la délivrance du brevet: **23.11.2016**
(21) Numéro de dépôt: 13820841.8
(22) Date de dépôt: 20.12.2013
(51) Int. Cl.: A61B 50/20, A61B 50/30, A61B 17/86

(54) **ENSEMBLE DU TYPE COMPRENANT AU MOINS UN IMPLANT, UN PREHENSEUR, ET UN ELEMENT DE TRAÇABILITE DE L'IMPLANT, ET PROCEDE DE MANIPULATION DE MANIERE TRAÇABLE DE L'IMPLANT D'UN TEL ENSEMBLE**
ANORDNUNG MIT MINDESTENS EINEM IMPLANTAT, EINER GREIFVORRICHTUNG UND EINEM ELEMENT ZUR VERFOLGUNG DES IMPLANTATS SOWIE VERFAHREN ZUR VERFOLGBAREN HANDHABUNG DES IMPLANTATS SOLCH EINER ANORDNUNG
ASSEMBLY INCLUDING AT LEAST ONE IMPLANT, ONE GRIPPING DEVICE AND ONE ELEMENT FOR THE TRACEABILITY OF THE IMPLANT, AND METHOD FOR TRACEABLY HANDLING THE IMPLANT OF SUCH AN ASSEMBLY

(30) Priorité: 31.12.2012 FR 1262987; 31.12.2012 FR 1262988
(43) Date de publication de la demande: 04.11.2015
(73) Titulaire: Neosteo, 44100 Nantes (FR)
(72) Inventeur: DEROUET, Guillaume, 44420 La Turballe (FR); DECHELETTE, Maxime, 44400 Rezé (FR)
(74) Mandataire: Godineau, Valérie
(86) Numéro de dépôt international: PCT/FR2013/053210
(87) Numéro de publication internationale: WO 2014/102491

(56) Documents cités:
- EP-A1- 2 392 286
- EP-B1- 1 241 998
- WO-A2-2009/024189
- DE-U1-202010 007 487
- FR-A1- 2 959 216
- US-A1- 2009 266 728

## Description

La présente invention concerne un ensemble du type comprenant au moins un implant, un préhenseur dudit implant, un élément de rangement du préhenseur et de son implant associé, et un élément de traçabilité de l'implant, ainsi qu'un procédé de manipulation de manière traçable de l'implant d'un tel ensemble.

Les ensembles du type précités sont bien connus à ceux versés dans cet art comme l'illustre en particulier le brevet EP 1.241.998, sur lequel est basée la forme en deux parties de la revendication 1.

Généralement, et comme l'illustre ce document de l'art antérieur, les moyens d'identification de l'implant sont disposés directement sur le préhenseur. Dans ce mode de réalisation, le chirurgien saisit directement le préhenseur associé à l'implant et stocké dans l'élément de rangement. Il implante l'implant dans le corps humain ou animal puis redonne le préhenseur à la personne chargée de la traçabilité. Celle-ci prend connaissance des informations mentionnées sur les préhenseurs et en général les reporte sur un support électronique ou papier. Il en résulte que le préhenseur qui a pu être potentiellement contaminé lors de l'implantation doit passer de mains en mains au risque de provoquer une dissémination de la contamination alors qu'il aurait dû aller directement à la poubelle.

Un ensemble de type comprenant au moins un implant, un préhenseur dudit implant, un élément de rangement du préhenseur et de son implant associé, et un élément de traçabilité de l'implant est également connu de la demande internationale WO 2009/024189. Enfin, d'autres ensembles comprenant un implant et un préhenseur dudit implant et un élément de rangement de préhenseur et de son implant sont décrits dans la demande de brevet américain US 2009/0266728 et la demande de brevet français FR 2.959.216.

Un but de la présente invention est donc de proposer un ensemble du type précité dont la conception permet d'éviter un transfert du préhenseur de mains en mains pour éviter toute dissémination d'une contamination éventuelle.

Un autre but de la présente invention est de proposer un ensemble dont la conception permet, si nécessaire, une manipulation de l'implant depuis l'élément de rangement jusqu'à son implantation dans le corps humain ou animal sans contact tactile avec l'implant.

A cet effet, l'invention a pour objet un ensemble du type comprenant au moins un implant, un préhenseur dudit implant, un élément de rangement du préhenseur et de son implant associé, ledit élément de rangement comprenant au moins un emplacement de réception du préhenseur et de son implant associé, un élément de traçabilité de l'implant, caractérisé en ce que l'implant présente une extrémité distale d'introduction de l'implant dans le corps humain ou animal et une extrémité proximale, appelée tête de l'implant, apte à coopérer avec un outil de manoeuvre, en ce que le préhenseur, séparable de l'implant, se présente sous forme d'un corps creux, allongé, ouvert à au moins l'une de ses extrémités, et fendu longitudinalement sur au moins une partie, de sa longueur, ce corps étant réalisé au moins partiellement en un matériau élastiquement déformable tendant à rappeler les bords de la fente dans le sens d'un rapprochement, cette fente dudit corps débouchant dans la cavité axiale dudit corps à l'intérieur de laquelle l'implant est apte à être inséré avec son extrémité distale faisant saillie de l'extrémité dite distale dudit corps, et en ce que l'élément de rangement est formé d'au moins un plateau muni d'au moins une cavité correspondant à un emplacement de réception d'un préhenseur pré-équipé de son implant et en ce que l'élément de traçabilité est pris en sandwich entre lé préhenseur et l'élément de rangement, à l'état rangé du préhenseur et de l'implant associé dans un emplacement de réception de l'élément de rangement.

Comme l'élément de traçabilité est distinct du préhenseur et de l'implant et indépendant de ces derniers, il en résulte la possibilité de manipuler le préhenseur et son implant associé et de transférer le préhenseur et son implant associé d'un emplacement à un autre sans avoir à transférer simultanément l'élément de traçabilité qui peut être pris en charge par une autre personne.

De préférence, l'élément de traçabilité affecte la forme d'une rondelle équipée de moyens d'identification du préhenseur.

De préférence, la rondelle présente une épaisseur comprise entre 0,05 et 3 mm.

De préférence, le préhenseur, séparable de l'implant, se présente sous forme d'un corps creux, allongé, ouvert à chacune de ses extrémités. De préférence, le préhenseur, séparable de l'implant, se présente sous forme d'un corps creux, allongé, fendu longitudinalement sur toute sa longueur.

Dans un premier mode de réalisation de l'invention, la cavité ou au moins l'une des cavités de l'élément de rangement correspondant à un emplacement de réception d'un préhenseur pré-équipé de son implant, présente un axe longitudinal sensiblement orthogonal à la surface dudit plateau, le corps du préhenseur, introduit axialement par son extrémité distale dans la cavité, est un corps tubulaire épaulé, ledit épaulement périphérique externe depuis l'extrémité distale en direction de l'extrémité proximale dudit corps formant une butée de limitation d'introduction du corps à l'intérieur dudit emplacement de réception de l'élément de rangement et l'élément de traçabilité est pris en sandwich entre la surface épaulée du préhenseur et la face supérieure du plateau.

Dans ce mode de réalisation, la rondelle présente un diamètre intérieur supérieur au diamètre extérieur du préhenseur dans la zone du préhenseur s'étendant entre extrémité distale et épaulement dudit préhenseur, et inférieur au diamètre extérieur du préhenseur dans la zone du préhenseur s'étendant entre extrémité proximale et épaulement dudit préhenseur.

La zone du préhenseur s'étendant entre extrémité distale et épaulement dudit préhenseur est logée au moins partiellement à emboîtement dans une cavité de l'élément de rangement avec la partie distale de l'implant, en saillie du préhenseur, s'étendant à l'intérieur de ladite cavité.

La tête de l'implant est, à l'état inséré de l'implant dans la cavité du préhenseur, écartée de l'extrémité proximale du préhenseur.

Dans un deuxième mode de réalisation de l'invention, la cavité ou au moins l'une des cavités, correspondant à un emplacement de réception d'un préhenseur pré-équipé de son implant, de l'élément de rangement présente un axe longitudinal sensiblement parallèle à la surface dudit plateau, en ce que le préhenseur et son implant s'étendent axialement à l'intérieur de la cavité et l'élément de traçabilité est pris en sandwich entre l'extrémité proximale du préhenseur et l'élément de rangement.

Indépendamment du mode de réalisation, la cavité axiale du corps du préhenseur comporte une zone de constriction de l'implant à l'intérieur de laquelle l'implant est apte à être retenu par serrage.

De préférence, ledit ensemble comprend en outre des moyens d'aide à la séparation du préhenseur de l'implant sans toucher l'implant, ces moyens comprenant au moins un fourreau de réception de l'extrémité distale de l'implant, l'implant étant, à l'état introduit de son extrémité distale dans le fourreau, séparable de son préhenseur par déplacement relatif du fourreau et du préhenseur dans le sens d'une rupture de l'alignement entre fourreau et préhenseur jusqu'à passage de l'implant à travers la fente du préhenseur, ledit fourreau étant muni d'un siège d'appui de l'implant à l'état introduit dudit implant dans le fourreau et à l'état séparé dudit implant de son préhenseur.

Dans un mode de réalisation préféré, le fourreau est monté solidaire de l'élément de rangement.

De préférence encore, au moins l'une des cavités de l'élément de rangement forme au moins une partie dudit fourreau.

Bien évidemment, le mode de réalisation dans lequel le fourreau est indépendant de l'élément de rangement entre également dans le champ de la présente invention.

L'invention a également pour objet un procédé pour la manipulation de manière traçable d'un implant d'un ensemble conforme à celui décrit ci-dessus, ledit implant et son préhenseur étant logés au moins partiellement à l'intérieur d'un emplacement de réception de l'élément de rangement, caractérisé en ce que ledit procédé comprend au moins une étape de saisie manuelle du préhenseur associé à l'implant jusqu'à extraction de l'implant de l'emplacement de réception et une étape de saisie manuelle de l'élément de traçabilité distinct et indépendant du préhenseur et de l'implant associé, cet élément de traçabilité étant disposé dans ou à l'entrée de l'emplacement de réception dudit élément de rangement.

Dans un mode de mise en oeuvre du procédé dans lequel l'ensemble comprend en outre des moyens d'aide à la séparation du préhenseur de l'implant sans toucher l'implant, ledit procédé comprend en outre une étape d'introduction d'au moins l'extrémité distale de l'implant saisi par son préhenseur dans ledit fourreau, une étape de séparation de l'implant de son préhenseur par déplacement relatif du fourreau et du préhenseur dans le sens d'une rupture de l'alignement entre fourreau et préhenseur jusqu'à passage de l'implant à travers la fente du préhenseur, une étape de retenue de l'implant dans le fourreau par appui sur le siège dudit fourreau jusqu'à saisie de l'implant par un outil de manoeuvre couplable de préférence à force avec la tête dudit implant.

L'invention sera bien comprise à la lecture de la description suivante d'exemples de réalisation, en référence aux dessins annexés dans lesquels :
Les figures 1 à 6 illustrent sous forme de vues en perspective les différentes étapes de manipulation d'un implant et du préhenseur associé ainsi que de l'élément de traçabilité ;

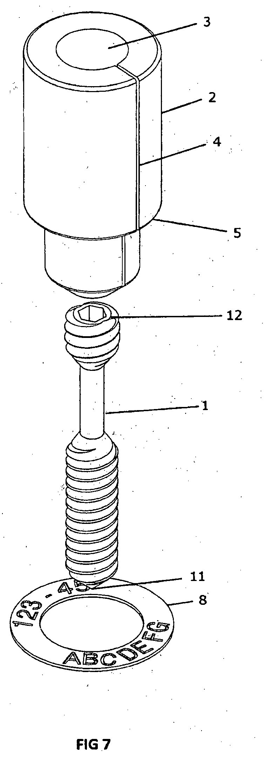

La figure 7 représente une vue en perspective du préhenseur, de l'implant et de l'élément de traçabilité d'un ensemble conforme à l'invention en position éclatée desdits éléments ;
La figure 8 représente une vue en coupe d'un élément de rangement comprenant deux cavités logeant chacune un implant associé à un préhenseur avec l'élément de traçabilité pris en sandwich entre le préhenseur et l'élément de rangement, et un fourreau logeant un implant à l'état séparé de son préhenseur et couplé à un outil de manoeuvre, tel qu'un tournevis ;
La figure 9 représente une vue en perspective d'un autre mode de réalisation d'un élément de rangement comprenant plusieurs cavités recevant chacune un préhenseur associé à un implant avec l'élément de traçabilité pris en sandwich entre préhenseur et élément de rangement ;
La figure 10 représente une vue en coupe de la figure 9.

Comme mentionné ci-dessus, l'ensemble, objet de l'invention, est plus particulièrement destiné à permettre la traçabilité d'implants 1 chirurgicaux, tels que des implants d'ostéosynthèse aptes à être implantés dans le corps humain ou animal.

Cet ensemble comprend donc un implant 1, un préhenseur 2 dudit implant, un élément 7 de rangement du préhenseur 2 et de son implant associé, ledit élément 7 de rangement comprenant au moins un emplacement 71 de réception du préhenseur 2 et de son implant associé, et un élément 8 de traçabilité dé l'implant. L'élément 8 de traçabilité de l'implant est indépendant et distinct du préhenseur 2 et de son implant associé de sorte que le préhenseur et son implant associé peuvent être sortis de l'emplacement de réception de l'élément de rangement et transférés en un autre emplacement sans transfert simultané de l'élément 8 de traçabilité.

Dans les exemples représentés, l'implant 1 de type vis présente une extrémité 11 distale d'introduction de l'implant 1 dans le corps humain ou animal et une extrémité 12 proximale, appelée tête de l'implant, apte à coopérer avec un outil 9 de manoeuvre.

Cette tête d'implant formée par la tête de la vis comporte une empreinte en creux à l'intérieur de laquelle l'extrémité de l'outil de manoeuvre, tel qu'un tournevis, est apte à être insérée à force pour permettre, à l'état couplé de l'implant et de l'outil de manoeuvre, à l'aide de l'outil de manoeuvre, l'entraînement en rotation de l'implant pour son implantation dans le corps humain ou animal.

Le préhenseur 2 séparable de l'implant 1 se présente, quant à lui ici, sous forme d'un corps 21 creux tubulaire ouvert à chacune de ses extrémités et fendu longitudinalement sur toute sa longueur. Ce corps 21 est réalisé au moins partiellement en un matériau élastiquement déformable, tel qu'un élastomère. Ce matériau tend à rappeler les bords de la fente 4 dans le sens d'un rapprochement.

Cette fente 4 du corps 21 débouche dans la cavité 3 axiale du corps 21 à l'intérieur de laquelle l'implant 1 est apte à être inséré avec son'extrémité 11 distale faisant saillie de l'extrémité distale dudit corps 21.

La cavité 3 axiale du corps du préhenseur 2 comporte une zone 31 de constriction de l'implant à l'intérieur de laquelle l'implant 1 est apte à être retenu par serrage.

La tête de l'implant est, à l'état inséré de l'implant dans la cavité du préhenseur, écartée de l'extrémité proximale du préhenseur pour éviter notamment tout contact tactile de l'implant avec la main du chirurgien lors de la saisie du préhenseur.

Dans les exemples représentés, le corps 21 tubulaire du préhenseur est un corps épaulé. Cet épaulement 5 forme un épaulement périphérique externe depuis l'extrémité distale en direction de l'extrémité proximale du corps 21 du préhenseur 2.

L'élément 7 de rangement est, quant à lui, formé d'un plateau muni d'au moins une cavité 71 correspondant à un emplacement de réception d'un préhenseur pré-équipé de son implant.

Ce plateau peut être relativement épais pour former le corps d'une boîte fermée par un couvercle. Ce plateau peut également être inséré dans un corps de boîte à nouveau, de préférence, fermée par un couvercle.

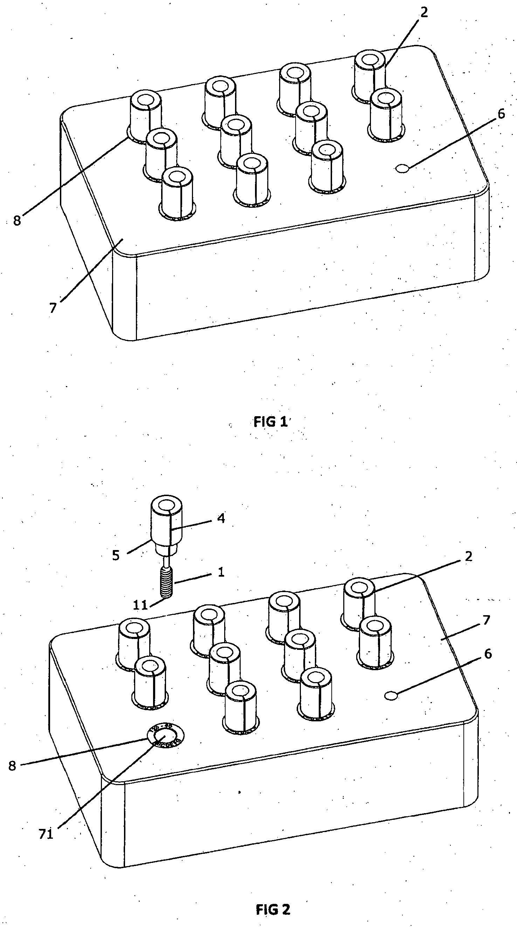

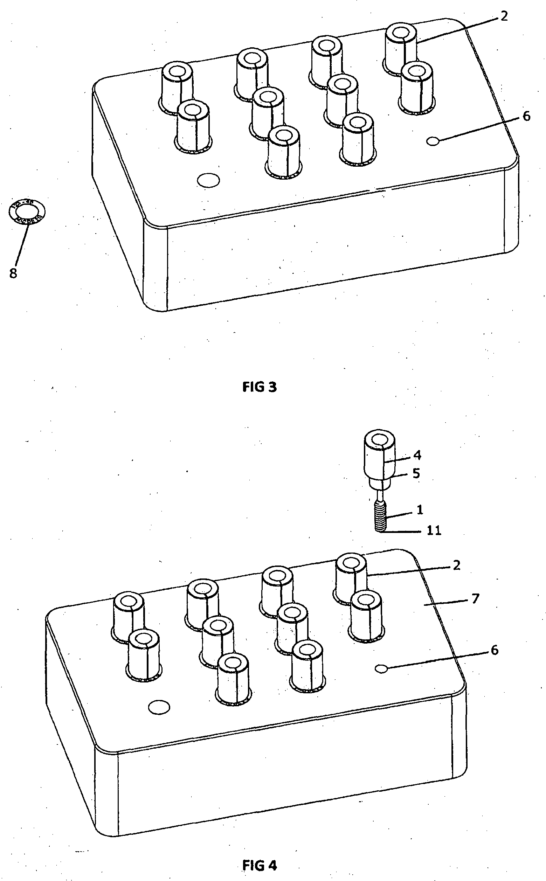

Dans les exemples représentés dans les figures 1 à 6, l'élément 7 de rangement comporte onze cavités 71 servant chacune à la réception d'un implant et de son préhenseur associé.

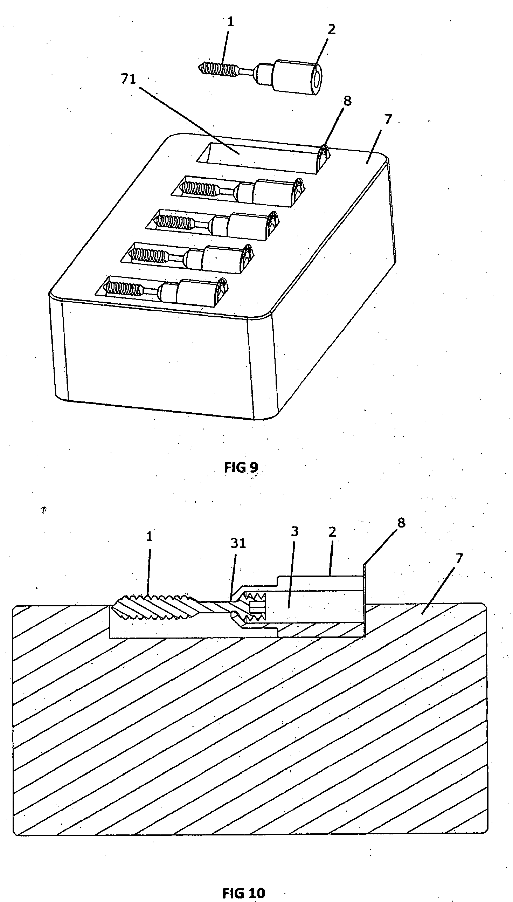

Dans l'exemple de réalisation des figures 9 et 10, l'élément 7 de rangement comporte cinq cavités 71.

Dans l'exemple représenté aux figures 1 à 6, chaque cavité est une cavité cylindrique d'axe longitudinal sensiblement perpendiculaire à la surface du plateau.

Le corps 21 du préhenseur 2 est introduit axialement par son extrémité distale dans une cavité 3 jusqu'à ce que l'épaulement 5 périphérique externe dudit corps forme une butée de limitation d'introduction du corps à l'intérieur dudit emplacement par venue en appui contre la face supérieure dudit plateau.

Dans ce mode de réalisation, la zone du préhenseur s'étendant entre extrémité distale et épaulement 5 du préhenseur 2 est logée au moins partiellement à emboîtement dans la cavité 71 de l'élément 7 de rangement avec la partie distale de l'implant 1 en saillie du préhenseur 2 s'étendant à l'intérieur de la cavité 71. Il en résulte que l'implant est complètement invisible depuis l'extérieur.

Dans l'exemple représenté aux figures 9 et 10, chaque cavité est une cavité hémicylindrique d'axe longitudinal sensiblement parallèle à la surface du plateau. Le préhenseur 2 et son implant 1 s'étendent axialement à l'intérieur de la cavité 71. Ils forment ainsi un ensemble couché à l'intérieur de ladite cavité.

L'ensemble comporte encore un élément 8 de traçabilité pris en sandwich entre le préhenseur 2 et l'élément 7 de rangement à l'état rangé du préhenseur 2 et de l'implant 1 associé dans une cavité 71 de réception de l'élément 7 de rangement.

Cet élément 8 de traçabilité affecte ici la forme d'une rondelle équipée de moyens d'identification du préhenseur 2. Cette rondelle comporte une épaisseur comprise entre 0,05 et 3 mm.

Dans l'exemple représenté aux figures 1 à 6, l'élément de traçabilité est pris en sandwich entre la surface épaulée du préhenseur 2 et la face supérieure du plateau.

La rondelle constitutive de cet élément de traçabilité présente un diamètre intérieur supérieur au diamètre extérieur du préhenseur 2 dans la zone du préhenseur s'étendant entre extrémité distale et épaulement 5 dudit préhenseur et inférieur au diamètre extérieur du préhenseur 2 dans la zone du préhenseur s'étendant entre extrémité proximale et épaulement 5 dudit préhenseur 2.

La rondelle entoure donc le corps du préhenseur dans la zone du préhenseur qui s'étend entre extrémité distale et épaulement 5 du préhenseur et est limitée en déplacement axial le long du corps du préhenseur par l'épaulement 5 dudit corps 21.

Grâce à cette configuration, l'élément de traçabilité est au moins partiellement masqué à l'état monté du préhenseur et de son implant associé dans une cavité de l'élément de rangement. Ainsi, tout risque d'une saisie simultanée de l'élément de traçabilité lors de la saisie du préhenseur est empêchée.

Dans l'exemple représenté aux figures 9 et 10, l'élément 8 de traçabilité est pris en sandwich entre l'extrémité proximale du préhenseur 2 et l'élément 7 de rangement, en l'occurrence, entre une face d'extrémité de la cavité hémicylindrique et l'extrémité proximale du préhenseur.

Indépendamment du mode de réalisation de l'élément de rangement, la manipulation s'opère comme suit :
L'implant est logé au moins partiellement avec son préhenseur dans l'élément de rangement (figure 1), le préhenseur est saisi par un opérateur pour extraire le préhenseur et l'implant associé de l'élément de rangement (figure 2). L'élément 8 de traçabilité reste en position autour de la cavité de l'élément de rangement qui vient d'être libérée de l'implant et du préhenseur associé. Cette manipulation est facilitée par le fait que la rondelle et le préhenseur sont réalisés en deux matériaux incompatibles pour ne pas générer une adhérence entre eux. Ainsi, par exemple, la rondelle est en métal, en particulier en inox, et le préhenseur est en silicone. Il n'y a donc aucune adhérence entre ces matériaux.

La rondelle de traçabilité est d'un diamètre intérieur largement supérieur à la cavité de l'élément de rangement accueillant le préhenseur et au diamètre extérieur du préhenseur dans la zone du préhenseur s'étendant entre l'extrémité distale et l'épaulement dudit préhenseur.

Comme le diamètre intérieur de cette rondelle est également inférieur au diamètre du préhenseur entre l'épaulement et l'extrémité proximale du préhenseur, la rondelle reste inaccessible tant que le préhenseur n'est pas retiré. Il est donc pratiquement impossible que l'opérateur décolle la rondelle en même temps que le préhenseur.

Le préhenseur ne peut en aucun cas s'expanser au-delà du diamètre de la cavité de l'élément de rangement et donc ne touche pas la rondelle lors de l'extraction.

La faible épaisseur de la rondelle interdit toute préhension de la rondelle. Pour être retirée.ou mise en place il faut obligatoirement retirer le préhenseur et son implant puis appuyer la rondelle sur la surface du plateau et la faire glisser pour pouvoir la mettre en place ou la retirer.

En conséquence, à aucun moment l'opérateur ne peut disposer à la fois du préhenseur associé à l'implant et de l'élément de traçabilité.

Dans le mode de réalisation représenté, une fois le préhenseur et son implant retirés de l'élément de rangement, la rondelle peut être saisie manuellement en vue d'une lecture des informations mentionnées sur ladite rondelle et éventuellement un report de ces informations sur un support quelconque.

Dans le mode de réalisation des figures 1 à 6, ledit ensemble comprend encore des moyens d'aide à la séparation du préhenseur de l'implant, de préférence sans toucher l'implant.

Ces moyens comprennent un fourreau 6 de réception de l'extrémité 11 distale de l'implant 1. Ce fourreau est monté solidaire de l'élément de rangement et est formé par une des cavités de l'élément de rangement. Cette cavité est de diamètre inférieur aux autres cavités. En particulier, cette cavité présente un diamètre inférieur au diamètre extérieur du préhenseur.

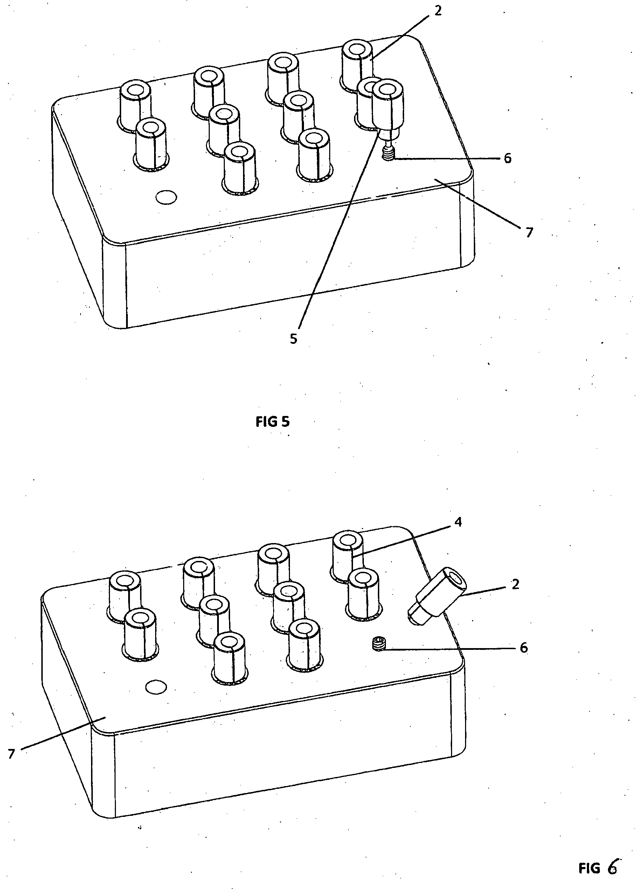

L'implant est, à l'état introduit de son extrémité 11 distale dans le fourreau 6, séparable de son préhenseur 2 par déplacement relatif du fourreau 6 et du préhenseur dans le sens d'une rupture de l'alignement entre fourreau 6 et préhenseur 2, comme l'illustre la figure 6, jusqu'à passe de l'implant 1 à travers la fente 4 du préhenseur 2.

Le fourreau 6 est muni d'un siège 61 d'appui de l'implant 1 à l'état introduit dudit implant 1 dans le fourreau 6 et à l'état séparé dudit implant 1 de son préhenseur. Ce siège est ici formé par l'extrémité libre du fourreau par laquelle l'implant est introduit à l'intérieur du fourreau.

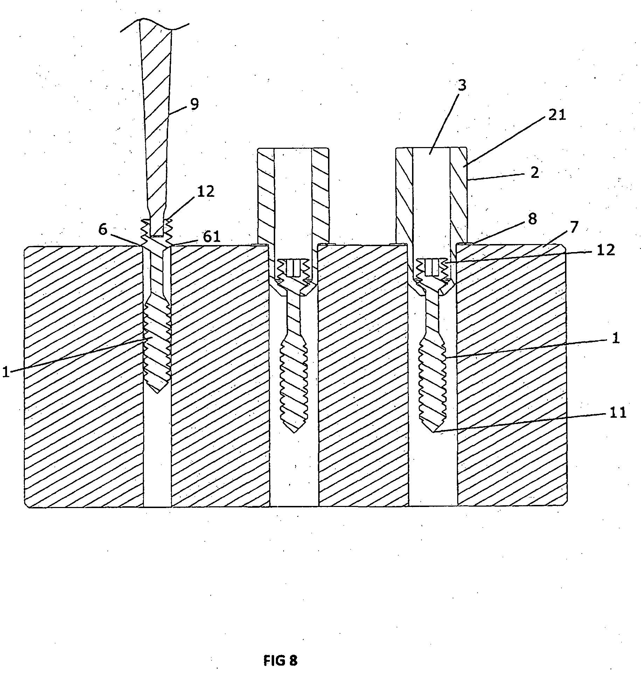

L'implant est donc retenu en butée dans le fourreau et peut être couplé à un outil 9 de manoeuvre, comme l'illustre la figure 8. Un tel couplage n'aurait pas pu être possible si l'implant était demeuré à l'intérieur du préhenseur en raison de la déformation élastique du préhenseur qui rend difficile tout couplage de l'implant et de l'outil de manoeuvre avec un risque élevé de couplage imparfait ou défaillant pouvant amener à la perte de l'implant ou à un mauvais entraînement en rotation de l'implant.

Une fois l'implant et son outil de manoeuvre couplés l'un à l'autre, l'implantation peut être opérée.

La procédure, telle que décrite ci-dessus, peut également s'appliquer à l'exemple de réalisation des figures 9 et 10.

La procédure, telle que décrite ci-dessus, montre qu'il est possible de transférer l'implant de son élément de rangement dans le corps humain ou animal sans, à aucun moment, que la main du chirurgien n'entre en contact avec ledit implant.

Il en résulte des risques de contamination moindres.

## Revendications

1. Ensemble du type comprenant au moins un implant (1), un préhenseur (2) dudit implant (1), un élément (7) de rangement du préhenseur (2) et de son implant (1) associé, ledit élément (7) de rangement comprenant au moins un emplacement (71) de réception du préhenseur (2) et de son implant (1) associé, un élément (8) de traçabilité de l'implant (1), l'implant (1) présentant une extrémité (11) distale d'introduction de l'implant (1) dans le corps humain ou animal et une extrémité (12) proximale, appelée tête de l'implant, apte à coopérer avec un outil (9) de manoeuvre, le préhenseur (2), séparable de l'implant (1), se présentant sous forme d'un corps (21) creux, allongé, ouvert à au moins l'une de ses extrémités,
**caractérisé en ce que** le corps (21) du préhenseur est fendu longitudinalement sur au moins une partie de sa longueur et réalisé au moins partiellement en un matériau élastiquement déformable tendant à rappeler les bords de la fente (4) dans le sens d'un rapprochement, cette fente (4) dudit corps (21) débouchant dans la cavité (3) axiale dudit corps (21) à l'intérieur de laquelle l'implant (1) est apte à être inséré avec son extrémité (11) distale faisant saillie de l'extrémité dite distale dudit corps (21), et **en ce que** l'élément (7) de rangement est formé d'au moins un plateau muni d'au moins une cavité (71) correspondant à un emplacement (71) de réception d'un préhenseur (2) pré-équipé de son implant (1) et **en ce que** l'élément (8) de traçabilité est pris en sandwich entre le préhenseur (2) et l'élément (7) de rangement à l'état rangé du préhenseur (2) et de l'implant (1) associé dans un emplacement (71) de réception de l'élément (7) de rangement.

2. Ensemble selon la revendication 1,
**caractérisé en ce que** l'élément (8) de traçabilité affecte la forme d'une rondelle équipée de moyens d'identification du préhenseur (2).

3. Ensemble selon la revendication 2,
**caractérisé en ce que** la rondelle présente une épaisseur comprise entre 0,05 et 3 mm.

4. Ensemble selon l'une des revendications précédentes,
**caractérisé en ce que** la cavité, ou au moins l'une des cavités de l'élément (7) de rangement, correspondant à un emplacement de réception d'un préhenseur (2) pré-équipé de son implant (1), présente un axe longitudinal sensiblement orthogonal à la surface dudit plateau, **en ce que** le corps (21) du préhenseur (2), introduit axialement par son extrémité distale dans la cavité (71), est un corps tubulaire épaulé, ledit épaulement (5) périphérique externe depuis l'extrémité distale en direction de l'extrémité proximale dudit corps (21) formant une butée de limitation d'introduction du corps (21) à l'intérieur dudit emplacement de réception de l'élément (7) de rangement et **en ce que** l'élément (8) de traçabilité est pris en sandwich entre la surface épaulée du préhenseur (2) et la face supérieure du plateau.

5. Ensemble selon l'une des revendications précédentes prise en combinaison avec la revendication 2,
**caractérisé en ce que** la rondelle présente un diamètre intérieur supérieur au diamètre extérieur du préhenseur dans la zone du préhenseur (2) s'étendant entre extrémité distale et épaulement (5) dudit préhenseur (2), et inférieur au diamètre extérieur du préhenseur (2) dans la zone du préhenseur s'étendant entre extrémité proximale et épaulement (5) dudit préhenseur (2).

6. Ensemble selon l'une des revendications précédentes,
**caractérisé en ce que** la zone du préhenseur s'étendant entre extrémité distale et épaulement (5) dudit préhenseur (2) est logée au moins partiellement à emboîtement dans une cavité (71) de l'élément (7) de rangement avec la partie distale de l'implant (1), en saillie du préhenseur (2), s'étendant à l'intérieur de ladite cavité (71).

7. Ensemble selon l'une des revendications 1 à 3,
**caractérisé en ce que** la cavité (7), ou au moins l'une des cavités de l'élément (7) de rangement, correspondant à un emplacement de réception d'un préhenseur (2) pré-équipé de son implant (1), présente un axe longitudinal sensiblement parallèle à la surface dudit plateau, **en ce que** le préhenseur (2) et son implant (1) s'étendent axialement à l'intérieur de la cavité (71) et **en ce que** l'élément (8) de traçabilité est pris en sandwich entre l'extrémité proximale du préhenseur (2) et l'élément (7) de rangement.

8. Ensemble selon l'une des revendications précédentes,
**caractérisé en ce que** ledit ensemble comprend en outre des moyens d'aide à la séparation du préhenseur de l'implant de préférence sans toucher l'implant, ces moyens comprenant au moins un fourreau (6) de réception de l'extrémité (11) distale de l'implant (1), l'implant (1) étant, à l'état introduit de son extrémité (11) distale dans le fourreau (6), séparable de son préhenseur (2) par déplacement relatif du fourreau (6) et du préhenseur (2) dans le sens d'une rupture de l'alignement entre fourreau (6) et préhenseur (2) jusqu'à passage de l'implant (1) à travers la fente (4) du préhenseur (2), ledit fourreau (6) étant muni d'un siège (61) d'appui de l'implant (1) à l'état introduit dudit implant (1) dans le fourreau (6) et à l'état séparé dudit implant (1) de son préhenseur (2).

9. Procédé pour la manipulation de manière traçable d'un implant (1) d'un ensemble conforme à l'une des revendications 1 à 8, ledit implant (1) et son préhenseur (2) étant logés au moins partiellement à l'intérieur d'un emplacement (71) de réception de l'élément (7) de rangement, **caractérisé en ce que** ledit procédé comprend au moins une étape de saisie manuelle du préhenseur (2) associé à l'implant (1) jusqu'à extraction de l'implant (1) de l'emplacement (71) de réception et une étape de saisie manuelle de l'élément (8) de traçabilité distinct et indépendant du préhenseur et de l'implant associé, cet élément de traçabilité étant disposé dans ou à l'entrée de l'emplacement (71) de réception dudit élément (7) de rangement.

## Patentansprüche

1. Anordnung der Bauart, umfassend mindestens ein Implantat (1), eine Greifvorrichtung (2) des Implantats (1), ein Verstauelement (7) der Greifvorrichtung (2) und ihres zugeordneten Implantats (1), wobei das Verstauelement (7) mindestens eine Empfangsstelle (71) der Greifvorrichtung (2) und ihres zugeordneten Implantats (1), ein Verfolgbarkeitselement (8) des Implantats (1) umfasst,
wobei das Implantat (1) ein distales Einführende (11) des Implantats (1) in den Körper eines Menschen oder eines Tiers und ein als Implantatkopf bezeichnetes proximales Ende (12), das imstande ist, mit einem Manövrierwerkzeug (9) zusammenzuarbeiten, aufweist, dass die vom Implantat (1) trennbare Greifvorrichtung (2) die Form eines länglichen Hohlkörpers (21) aufweist, der an mindestens einem ihrer Enden offen ist, **dadurch gekennzeichnet, dass** der Körper (21) Greifvorrichtung über mindestens einen Teil ihrer Länge geschlitzt ist, und mindestens teilweise aus einem elastisch verformbaren Material hergestellt ist, das dazu tendiert, die Ränder des Schlitzes (4) in Richtung einer Annäherung zurückzustellen, wobei dieser Schlitz (4) des Körpers (21) in den axialen Hohlraum (3) des Körpers (21) mündet, in dessen Innern das Implantat (1) imstande ist, mit seinem distalen Ende (11), das aus dem distalen Ende des Körpers (21) hervorsteht, eingesetzt zu sein, und dass das Verstauelement (7) von mindestens einer Platte, ausgestattet mit mindestens einem entsprechenden Hohlraum (71), der einer Empfangsstelle (71) einer mit ihrem Implantat (1) vorausgestatteten Greifvorrichtung (2) entspricht, gebildet ist und dass das Verfolgbarkeitselement (8) zwischen der Greifvorrichtung (2) und dem Verstauelement (7) im verstauten Zustand der Greifvorrichtung (2) und des zugeordneten Implantats (1) in einer Empfangsstelle (71) des Verstauelements (7) sandwichartig eingeschlossen ist.

2. Anordnung nach Anspruch 1,
**dadurch gekennzeichnet, dass** das Verfolgbarkeitselement (8) die Form einer Scheibe hat, die mit Identifizierungsmitteln der Greifvorrichtung (2) ausgestattet ist.

3. Anordnung nach Anspruch 2,
**dadurch gekennzeichnet, dass** die Scheibe eine Dicke zwischen 0,05 und 3 mm inklusive hat.

4. Anordnung nach einem der vorangehenden Ansprüche,
**dadurch gekennzeichnet, dass** der Hohlraum oder mindestens einer der Hohlräume des Verstauelements (7), der einer Empfangsstelle einer mit ihrem Implantat (1) vorausgestatteten Greifvorrichtung (2) entspricht, eine zur Oberfläche der Platte etwa orthogonale Längsachse aufweist, dass der Körper (21) der Greifvorrichtung (2), der mittels seines distalen Endes axial in den Hohlraum (71) eingeführt ist, ein rohrförmiger Körper mit Absatz ist, wobei dieser äußere Umfangsabsatz (5) ab dem distalen Ende in Richtung des proximalen Endes des Körpers (21) einen Begrenzungsanschlag für das Einsetzen des Körpers (21) in das Innere der Empfangsstelle des Verstauelements (7) bildet und dass das Verfolgbarkeitselement (8) zwischen der Oberfläche mit Absatz der Greifvorrichtung (2) und der oberen Fläche der Platte sandwichartig eingeschlossen ist.

5. Anordnung nach einem der vorangehenden Ansprüche, herangezogen in Kombination mit Anspruch 2,
**dadurch gekennzeichnet, dass** die Scheibe einen Durchmesser aufweist, der größer als der Außendurchmesser der Greifvorrichtung in der Zone der Greifvorrichtung (2) ist, die sich zwischen dem distalen Ende und dem Absatz (5) der Greifvorrichtung (2) erstreckt, und kleiner als der Außendurchmesser der Greifvorrichtung (2) in der Zone der Greifvorrichtung, die sich zwischen dem proximalen Ende und dem Absatz (5) der Greifvorrichtung (2) erstreckt.

6. Anordnung nach einem der vorangehenden Ansprüche,
**dadurch gekennzeichnet, dass** die Zone der Greifvorrichtung, die sich zwischen dem distalen Ende und dem Absatz (5) der Greifvorrichtung (2) erstreckt, mindestens teilweise in einen Hohlraum (71) des Verstauelements (7) mit dem distalen Teil des Implantats (1), das aus der Greifvorrichtung (2) hervorsteht, eingerastet ist, das sich im Innern des Hohlraums (71) erstreckt.

7. Anordnung nach einem der Ansprüche 1 bis 3,
**dadurch gekennzeichnet, dass** der Hohlraum (7) oder mindestens einer der Hohlräume des Verstauelements (7), der einer Empfangsstelle einer mit ihrem Implantat (1) vorausgestatteten Greifvorrichtung (2) entspricht, eine zur Oberfläche der Platte etwa parallele Längsachse aufweist, dass sich die Greifvorrichtung (2) und ihr Implantat (1) axial im Innern des Hohlraums (71) erstrecken und dass das Verfolgbarkeitselement (8) zwischen dem proximalen Ende der Greifvorrichtung (2) und dem Verstauelement (7) sandwichartig eingeschlossen ist.

8. Anordnung nach einem der vorangehenden Ansprüche,
**dadurch gekennzeichnet, dass** die Anordnung ferner Hilfsmittel für das Trennen der Greifvorrichtung vom Implantat umfasst, vorzugsweise ohne das Implantat zu berühren, wobei diese Mittel mindestens eine Empfangshülse (6) des distalen Endes (11) des Implantats (1) umfassen, wobei das Implantat (1) im mit seinem distalen Ende (11) in die Hülse (6) eingesetzten Zustand von seiner Greifvorrichtung (2) mittels relativer Verlagerung der Hülse (6) und der Greifvorrichtung (2) in Richtung eines Verlassens der Flucht zwischen Hülse (6) und Greifvorrichtung (2) bis zum Durchgang des Implantats (1) durch den Schlitz (4) der Greifvorrichtung (2) trennbar ist, wobei die Hülse (6) im eingesetzten Zustand des Implantats (1) in die Hülse (6) und im getrennten Zustand des Implantats (1) von seiner Greifvorrichtung (2) mit einem Stützsitz (61) des Implantats (1) ausgestattet ist.

9. Verfahren für die verfolgbare Handhabung eines Implantats (1) einer Anordnung nach einem der Ansprüche 1 bis 9, wobei das Implantat (1) und seine Greifvorrichtung (2) mindestens teilweise im Innern einer Empfangsstelle (71) des Verstauelements (7) untergebracht sind, **dadurch gekennzeichnet, dass** das Verfahren mindestens einen manuellen Greifschritt der dem Implantat (1) zugeordneten Greifvorrichtung (2) bis zur Entnahme des Implantats (1) aus der Empfangsstelle (71) und einen manuellen Greifschritt des von der Greifvorrichtung und dem zugeordneten Implantat unterschiedlichen und unabhängigen Verfolgbarkeitselements (8) umfasst, wobei dieses Verfolgbarkeitselement in der oder am Eingang zur Empfangsstelle (71) des Verstauelements (7) angeordnet ist.

## Claims

1. An assembly comprising at least one implant (1), one gripping device (2) of said implant (1), a storage element (7) for the gripping device (2) and its associated implant (1), said storage element (7) comprising at least one location (71) for receiving the gripping device (2) and its associated implant (1), one element (8) for the traceability of the implant (1); the implant (1) having a distal end (11) for insertion of the implant (1) into the human or animal body and a proximal end (12), called head of the implant, able to cooperate with a maneuvering tool (9), the gripping device (2), separable from the implant (1), assumes the form of an elongate hollow body (21), open at least at one of its ends;
**characterized in that** the body of the gripping device has a longitudinal slit over at least part of its length and is at least partially made from an elastically deformable material tending to return the edges of the slit (4) in the direction coming closer together, this slit (4) of said body (21) emerging in the axial cavity (3) of said body (21) inside which the implant (1) is able to be inserted with its distal end (11) protruding from the so-called distal end of said body (21), and **in that** the storage element (7) is formed by at least one plate provided with at least one cavity (71) corresponding to a location (71) for receiving a gripping device (2) pre-equipped with its implant (1) and **in that** the traceability element (8) is sandwiched between the gripping device (2) and storage element (7) in the stored state of the gripping device (2) and the associated implant (1) in a location (71) for receiving the storage element (7).

2. The assembly according to claim 1,
**characterized in that** the traceability element (8) assumes the form of a washer equipped with means for identifying the gripping device (2).

3. The assembly according to claim 2,
**characterized in that** the washer has a thickness comprised between 0.05 and 3 mm.

4. The assembly according to one of the preceding claims,
**characterized in that** the cavity, or at least one of the cavities of the storage element (7), corresponding to a receiving location for a gripping device (2) pre-equipped with its implant (1), has a longitudinal axis substantially orthogonal to the surface of said plate, **in that** the body (21) of the gripping device (2), introduced axially by its distal end in the cavity (71), is a stepped tubular body, said outer peripheral shoulder (5) from the distal end toward the proximal end of said body (21) forming a stop limiting the insertion of the body (21) inside said receiving location of the storage element (7), and **in that** the traceability element (8) is sandwiched between the stepped surface of the gripping device (2) and the upper surface of the plate.

5. The assembly according to one of the preceding claims in combination with claim 2, **characterized in that** the washer has an inner diameter larger than the outer diameter of the gripping device in the zone of the gripping device (2) extending between the distal end and shoulder (5) of said gripping device (2), and smaller than the outer diameter of the gripping device (2) in the zone of the gripping device extending between the proximal end and shoulder (5) of said gripping device (2).

6. The assembly according to one of the preceding claims,
**characterized in that** the zone of the gripping device extending between distal end and shoulder (5) of the gripping device (2) is at least partially housed nested in a cavity (71) of the storage element (7) with the distal part of the implant (1), protruding from the gripping device (2), extending inside said cavity (71).

7. The assembly according to one of claims 1 to 3,
**characterized in that** the cavity (7), or at least one of the cavities of the storage element (7), corresponding to a location for receiving a gripping device (2) pre-equipped with its implant (1), has a longitudinal axis substantially parallel to the surface of said plate, **in that** the gripping device (2) and its implant (1) extend axially inside the cavity (71) and **in that** the traceability element (8) is sandwiched between the proximal end of the gripping device (2) and the storage element (7).

8. The assembly according to one of the preceding claims,
**characterized in that** said assembly further comprises means to assist with separating the gripping device from the implant preferably without touching the implant, these means comprising at least one sheath (6) for receiving the distal end (11) of the implant (1), the implant (1) being, in the state with its distal end (11) inserted into the sheath (6), separable from its gripping device (2) by relative movement of the sheath (6) and the gripping device (2) in the direction of a break in the alignment between the sheath (6) and the gripping device (2) until the implant (1) passes through the slit (4) of the gripping device (2), said sheath (6) being provided with a bearing seat (61) of the implant (1) in the state of said implant (1) inserted in the sheath (6) and in the state of said implant (1) separated from its gripping device (2).

9. A method for traceably manipulating an implant (1) of an assembly according to one of claims 1 to 8, said implant (1) and its gripping device (2) being at least partially housed inside a receiving location (71) of the storage element (7), **characterized in that** said method comprises at least one step for manually grasping the gripping member (2) associated with the implant (1) until the implant (1) is removed from the receiving location (71) and a step for manually grasping the traceability element (8) separate and independent from the gripping device and the associated implant, this traceability element being arranged in or at the inlet of the receiving location (71) of said storage element (7).
